# EUROPEAN PATENT APPLICATION

(11) **EP 3 714 828 A1**
(43) Date of publication of application: **30.09.2020**
(21) Application number: 20163707.1
(22) Date of filing: 17.03.2020
(51) Int. Cl.: A61B 18/18

(54) **PULMONARY DENERVATION WITH BRONCHIAL-CENTERED DIELECTRIC HEATING ELEMENT**

(30) Priority: 25.03.2019 US 201962823047 P; 05.03.2020 US 202016810039
(71) Applicant: Covidien LP, Mansfield, MA 02048 (US)
(72) Inventor: BRANNAN, Joseph D., Lyons, CO 80540 (US); LENTZ, Linnea R., NE Stacy, MN 55079 (US); LADTKOW, Casey M., Erie, CO 80516 (US); YANG, Zhongping, Woodbury, MN 55129 (US)
(74) Representative: Maschio, Antonio

(57) **Abstract**

A microwave ablation device including a catheter having an inner tube defining an inflow lumen therethrough and an outflow lumen between the inner tube and an inner surface of the catheter, a balloon in fluid communication with the inflow and outflow lumens, a microwave ablation antenna insertable into one of the inflow or outflow lumens, and a temperature sensor for detecting the temperature of at least one of a fluid circulating in the catheter or an airway wall against which a portion of the catheter is positioned.

## Description

### CROSS-REFERENCE TO RELATED APPLICATION

This application claims the benefit of and priority to U.S. Provisional Patent Application Serial No. 62/823,047, filed March 25, 2019, the entire disclosure of which is incorporated by reference herein.

### BACKGROUND

Bronchitis is the result of inflammation of the airways of the lungs that results in narrowing of the airways as well as the secretion of thick mucus, or phlegm, which builds-up and clogs the small airways in the bronchioles. This mucus build-up leads to symptoms such as coughing, wheezing, and shortness of breath. Often this mucus production is part of an inflammatory response caused by injury to the airways from smoking and other inhaled antagonists. The mucus can be so excessive that it overcomes the ability of the cilia within the lungs to sweep the mucus out and allow it to be expelled. Further, the mucus limits the size of the airways through which air must travel in the lungs, thus limiting the volume of air that can be inhaled. The combined effect causes a sufferer to persistently cough in a futile attempt to clear the mucus. This mucus can be so excessive that as it is drawn further and further distal in the lungs (e.g., to the alveoli which might not themselves be inflamed) the mucus limits the gas exchange as it coats the alveoli walls. The mucus reaching the alveoli further exacerbate the challenges of gas transfer experienced by smokers, where tar and other contaminates may already be covering the lining of the alveoli creating a barrier for gas exchange. Further, the mucus and other contaminants are a breeding ground for bacterial growth, further infection and even greater bronchitis symptoms. The classic description of someone suffering from chronic bronchitis is a "blue bloater." The color refers to the lack of oxygen successfully transferring from the alveoli to the blood stream and CO₂ being expelled from the blood stream through the alveoli to the atmosphere. These patients often appear bloated due obesity as well as water retention as a result of their compromised pulmonary and circulatory functions. As will be appreciated, many if not most patients will suffer from both emphysema issues and chronic bronchitis issues.

Fully functioning alveoli can often adapt and at least partially compensate for the reduction in total lung capacity caused by emphysema COPD. Indeed, this is one reason for the use of the highly invasive Lung Volume Reduction Surgery (LVRS) where wedges of damaged lung are removed to allow the remaining tissue to function better. In part this improved performance is enabled by the increase in space afforded the remaining alveoli to expand when the damaged portions of the lung are removed. By reducing the lung size, the remaining lung and surrounding muscles (intercostal and diaphragm) are able to work more efficiently. This makes breathing easier and helps patients achieve greater quality of life.

Aside from the highly invasive LVRS, the standard of care for lung diseases, such as asthma and bronchitis, has been focused largely on pharmaceutical treatment modalities such as bronchodilation and anti-inflammatory treatments. For example, a bronchodilator available under the brand name ADVAIR® is currently marketed by GlaxoSmithKline plc. for the treatment of COPD. Alternatively, it has been reported for decades that lung denervation via invasive means (e.g., surgery) may provide therapeutic benefit for asthma or emphysema. Again, such surgical treatment is invasive and results in the disablement of whole or parts of functions of the nerve that affects contraction of the damaged alveoli.

While these treatment options are effective to a point, the primary prescription for patients suffering from COPD is simply the administration of oxygen. Oxygen can alleviate some symptoms but does nothing to treat the underlying diseases.

### SUMMARY

The disclosure is directed to a microwave ablation device including a catheter having an inner tube defining an inflow lumen therein and an outflow lumen between the inner tube and an inner surface of the catheter, a balloon disposed on a distal portion of the catheter and in fluid communication with the inflow and outflow lumens, and a microwave ablation antenna, insertable into one of the inflow or outflow lumens. The microwave ablation device also includes at least one temperature sensor for detecting the temperature of at least one of a fluid circulating in the catheter or an airway wall against which a portion of the catheter is positioned.

The microwave ablation device of may further include at least one inflow port in fluid communication with the inflow lumen and for receiving inflow of a fluid. Additionally, or alternatively, the microwave ablation device may further include at least one outflow port in fluid communication with the outflow lumen and for allowing the outflow a fluid. Further, the microwave ablation device may include a port for receiving the microwave ablation antenna and limiting the loss of any fluid in the catheter.

In accordance with an aspect of the disclosure inflation of the balloon is achieved by the fluid introduced therein. The microwave ablation device may also include at least one position sensor operably coupled to at least one of a distal portion of the catheter or the balloon, which may be an electromagnetic sensor.

A further aspect of the disclosure is directed to a system including a catheter including an inner tube defining an inflow lumen therein and an outflow lumen between the inner tube and an inner surface of the catheter, a balloon disposed on a distal portion of the catheter and in fluid communication with the inflow and outflow lumens, a microwave ablation antenna insertable into the catheter for placement within the balloon, and a microwave generator in electrical communication with the microwave ablation antenna.

The system may include at least one temperature sensor for detecting the temperature of at least one of a fluid circulating in the catheter or an airway wall against which a portion of the catheter is positioned. Wherein upon detecting a predetermined temperature of the fluid within the balloon or a predetermined rate of change of the temperature of the fluid the generator is switched off. The system may also include an algorithm for driving the microwave generator to achieve a desired temperature profile of the fluid in the balloon or a desired temperature profile of an airway wall against which a portion of the catheter is positioned to limit damage to an airway in which the catheter is placed.

In a further aspect of the disclosure, the system includes at least one electromagnetic sensor secured to the catheter. The system may also include an electromagnetic navigation system for detecting a position of the electromagnetic sensor. The electromagnetic navigation system permits placement of the catheter and microwave ablation antenna proximate a desired location within the lungs of a patient such that application of energy results in severing of the nerve and denervation of the tissue at the desired location.

In yet another aspect of the disclosure, a system includes a microwave ablation antenna and a catheter configured to receive the microwave ablation antenna. The catheter includes an inner tube, a balloon, and a temperature sensor operably coupled to the balloon. The inner tube defines an inflow lumen and an outflow lumen. The outflow lumen is defined between the inner tube and an inner surface of the catheter. The balloon is disposed on a distal portion of the catheter and is in fluid communication with the inflow and outflow lumens. The balloon is configured to receive a fluid and surround a distal portion of the microwave ablation antenna when the catheter receives the microwave ablation antenna. The temperature sensor is configured to detect a temperature of at least one of the fluid received by the balloon or an airway wall against which a portion of the balloon is positioned.

In an aspect, the system includes a microwave generator configured to deliver microwave energy to the microwave ablation antenna.

In an aspect, the microwave generator is configured to be driven by an algorithm to achieve a desired temperature profile of the fluid received by the balloon or a desired temperature profile of the airway wall against which a portion of the balloon is positioned to limit damage to an airway in which the catheter is placed.

In an aspect, at least one electromagnetic sensor is operably coupled to at least one of the microwave ablation antenna or the catheter. The system may further include an electromagnetic navigation system configured to detect a position of the electromagnetic sensor.

### BRIEF DESCRIPTION OF THE DRAWINGS

FIG. 1 depicts a catheter in accordance with the disclosure inserted into the lungs of a patient;
FIG. 2 depicts a cross sectional view of a catheter in accordance with the disclosure inserted into the lungs of a patient; and
FIG. 3 depicts an electromagnetic navigation system and microwave ablation system in accordance with the disclosure.

### DETAILED DESCRIPTION

The disclosure relates to systems, and methods for performing medical intervention to treat lung deficiencies as a result of lung diseases. An example of such diseases is Chronic Obstructive Pulmonary Disorder (COPD) and particularly its two primary manifestations, emphysema and chronic bronchitis. More particularly, the disclosure relates to a system and method for enhanced navigation of a catheter and deployment of one or more energy application tools for the denervation of nerves affecting the afflicted tissue.

Additional treatment options are needed to increase the range of patients eligible to receive treatment and provide treatment options that yield a better result. The disclosure is directed to devices and methods for application of energy to and through an airway wall to denervate the tissue proximate diseased region of the lungs. In one embodiment of the disclosure a catheter having a balloon at a distal end portion is employed. A microwave ablation antenna is placed within the catheter and a radiating portion of the ablation antenna is centered within the balloon and/or aligned with the balloon. Once placed in the proper location within the airways of the patient, the balloon is filled with saline or de-ionized water. The ablation antenna is then energized until sufficient energy has been absorbed by the tissue surrounding the balloon and the radiating portion to ensure that the desired nerves have been severed.

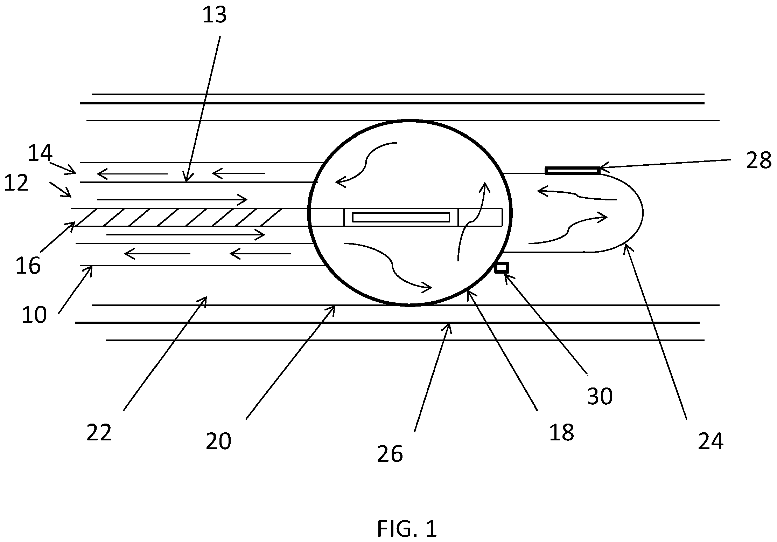

FIG. 1 depicts a catheter 10 in accordance with the disclosure. The catheter 10 in this embodiment includes an inner tube 13 defining an inner lumen 12 therethrough and an outer lumen 14 between the inner tube 13 and an inner surface of the catheter 10. As shown, the inner lumen 12 receives the ablation antenna 16 and also the inflow of a fluid such as saline or de-ionized, water which performs a variety of functions. The fluid (e.g., saline) is used to inflate the balloon 18 and secure the catheter 10 against the walls 20 of the airway 22. The fluid circulates within the balloon 18 and to the distal end 24 of the catheter. This circulation of fluid ensures that the balloon 18 and the tissue closest to the balloon 18 are not heated above a desired temperature. The desired temperature is preferably below the temperature at which tissue is desiccated, generally about 40° C.

Because microwave radiation applies energy to all areas through which it radiates and where it is absorbed, the cooling of the airway wall does not eliminate the heating effects of the microwave energy beyond the airway wall 20. That is, boundary cooling of the airway wall 20 does not prevent heating from occurring deeper within the tissue forming the airway 22. In effect, the cooling allows for projection of the treatment beyond the airway wall 20 to tissues beyond the airway wall such as the nerves 26 which run generally parallel to the airway wall 20. This projection is quite beneficial in preventing damage to the airway wall 20 which if injured can become locations of infection for these already compromised patients.

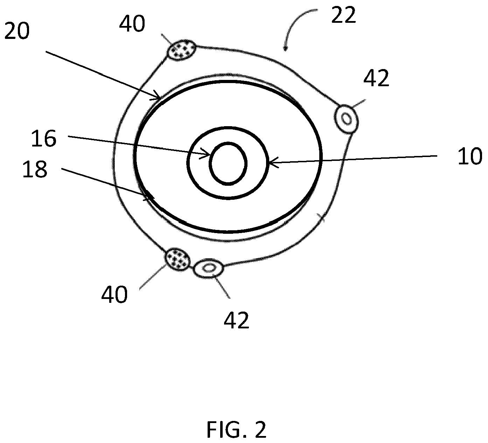

FIG. 2 depicts the interior of an airway 22 with the catheter 10 placed therein and the balloon 18 inflated with fluid (e.g., saline). The catheter 10 presses against the airway wall 20. The better the seal by the balloon 18 against the airway wall 20, the better the transfer of energy from the microwave ablation antenna 16, through the fluid and to the tissue. As is well known, therapeutic microwave energy does not pass through air particularly well, so it is desired that some care be taken to ensure a good seal of the balloon 18 against the airway wall 20.

The nerves 40 depicted on the airway 22 typically run along the airway 22. Though depicted on an exterior surface of the airway 22, those of skill in the art will appreciate that additional tissues may surround the airway 22 and are not shown here for ease of description. Also shown in FIG. 2 are blood vessels 42, which also may run generally parallel along the airway 22. As noted above, it is desirable that the lining of the airway and airway wall are not excessively heated by the microwave ablation antenna 16. In large part this is prevented by the circulation of the fluid within the balloon during the treatment process. This circulation actively cools the airway wall 20. Additionally, physiology assists in ensuring that the desired tissue is treated. As an initial matter blood vessels are themselves actively cooled by the passage of blood there through. The heat absorbed by the blood is carried off via circulation and is generally absorbed by the rest of the body without significantly altering the body temperature, and more importantly not resulting in ablation of the blood vessel or the areas around the blood vessel. Further, nerve tissue tends to necrose at temperatures slightly less than other tissues, particularly the tissue of the airway wall.

As a result of both the active cooling and physiology, it is possible for the microwave ablation antenna 16, when placed within the airway 22 with the balloon 18 inflated with fluid (e.g., saline), to circumferentially treat (e.g., radiate 360° from the microwave ablation antenna 16 and treat just the nerves 40, while not allowing the airway 22 or the blood vessels 42 to be ablated). As will be appreciated, a variety of "windowed" or limited radiation pattern microwave ablation antennas may also be used without departing from the scope of the disclosure in order to limit and/or focus the treatment or denervation effects to a particular area.

To assist in ensuring the nerves 40 are treated and not other tissues, the generator 122, described in greater detail below, may employ a variety of functions to manage the energy application. This may include a series of ramp and pulse functions to increase the energy applied to the airway until a desired temperature profile is achieved which ensures the nerve is severed or denervated, but other tissues are not unnecessarily harmed. Part of the function or energy algorithm may involve monitoring the airway wall 20 temperature or rates of change of the airway wall 20 temperature as energy is applied.

The catheter 10 may include multiple ports on a proximal end thereof. Two of the ports may be associated with a central lumen (e.g., inner lumen 12). A proximal port may include a luer connection for attachment of a saline bag or other fluid source. The distal port associated with the central lumen may include a slip connection. The slip connection allows for microwave ablation antenna 16 to be inserted into the central lumen and to be fed through an opening in the port. An elastomeric inner liner of the slip port prevents the fluid from exiting the port by forming a seal around the microwave ablation antenna 16. Upon reaching the desired location, the slip connection is rotated to lock the microwave ablation antenna 16 in place. The fluid flow through the central lumen can be begun before or after the insertion of the microwave ablation antenna 16. A third port may also include a luer connection for attachment to a waste receptacle or connection back to the fluid source (e.g., a saline drip bag). The ports associated with inflow and outflow of fluid may include pressure regulating apertures, which may be opened and closed to varying degrees to control flow and back pressure within the fluid flow pathway. With control of these pressure regulating apertures, either automated or manual, the status of balloon inflation can be modulated. During the initial placement of the balloon, its deployment may be achieved by creating back pressure in the system. After treatment, or between placements within the airways, the flow direction through the system may be reversed to deflate the balloon. In some instances, a peristaltic pump may be employed to ensure flow of fluid from the fluid source through the catheter.

The balloon 18 may include one or more of a variety of temperature sensors 28 (e.g., thermocouples) attached to the exterior or interior surface of the balloon 18. These temperature sensor(s) 28 provide an indication of the temperature of the airway wall and/or the temperature of the fluid within the balloon 18 or other portions of the catheter 10. The temperature sensor(s) 28 may be operably connected to a microwave generator. In operation, upon detecting a temperature or a rate of change of a temperature in excess of a set point, the generator is controlled to prevent overheating and damage to the airway wall. In an aspect, upon detecting a temperature or a rate of change of a temperature in excess of a set point, the generator is switched off to prevent overheating and damage to the airway wall. In another aspect, upon detecting a temperature or a rate of change of a temperature in excess of a set point, the output of the generator is reduced to prevent overheating and damage to the airway wall.

In accordance with one aspect of the disclosure, the catheter 10 may be placed in the lung using one or more visualization techniques including ultrasound imaging, fluoroscopy, CT imaging, and others to ensure proper placement. In such a scenario, the patient is intubated and the catheter 10 is navigated through the lungs, potentially following a guide wire placed via bronchoscopy or other techniques.

Another feature of the balloon 18 are one or more position sensors 30 (e.g., electromagnetic sensors) which may be placed in one or more locations on the catheter 10 and particularly the balloon 18. For example, one sensor 30 may be disposed on a distal end 24 of the catheter 10 to determine the extent of depth of insertion of the catheter 10. One or more further sensors may be used to determine placement of the balloon 18 relative to the microwave ablation antenna 16, to assess whether the balloon 18 has rotated in the airway, or to determine balloon 18 inflation status (e.g., a volume, roundness, etc. of the balloon 18).

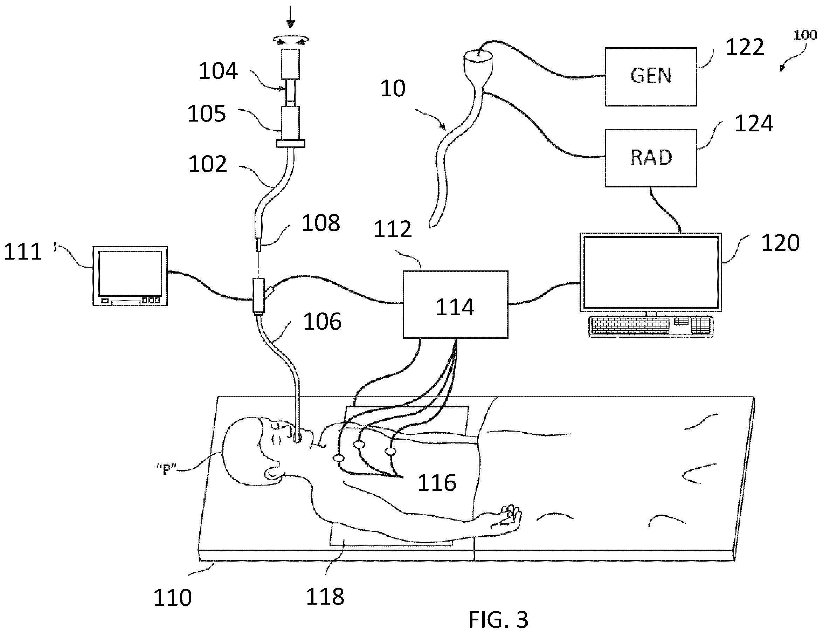

FIG. 3 depicts an Electromagnetic Navigation (EMN) system 100 configured for reviewing CT image data (such as that described above with respect to hypodensity and vascular tree identification) to identify one or more targets, planning a pathway to an identified target (planning phase), navigating an extended working channel (EWC) 102 to the target (navigation phase), and confirming placement of the EWC 102 within the target. One such EMN system is the ELECTROMAGNETIC NAVIGATION BRONCHOSCOPY® system currently sold by Medtronic pic.

As shown in FIG. 3, extended working channel 102 is part of a catheter guide assembly 104. In practice, the extended working channel 102 is inserted into bronchoscope 106 for access to a luminal network of the patient "P." Specifically, EWC 102 of catheter guide assembly 104 may be inserted into a working channel of bronchoscope 106 for navigation through a patient's luminal network and maneuvered using a telescoping handle 105. The EWC 102 may include a sensor 108, or another component inserted into the EWC 102 may include a sensor 108. This other component may be inserted into the extended working channel 102 and locked into position such that the sensor 108 extends a desired distance beyond the distal tip of the extended working channel 102. The position and orientation (6 DOF) of the sensor 108 relative to the reference coordinate system, and thus the distal end of the extended working channel 102, within an electromagnetic field can be derived. Catheter guide assemblies 104 are currently marketed and sold by Medtronic under the name SUPERDIMENSION® Procedure Kits, or EDGE™ Procedure Kits, and are contemplated as useable with the disclosure.

System 100 generally includes an operating table 110 configured to support a patient "P;" a bronchoscope 106 configured for insertion through the patient's "P's" mouth into the patient's "P's" airways; monitoring equipment 111 coupled to bronchoscope 106 (e.g., a video display, for displaying the video images received from the video imaging system of bronchoscope 106); a tracking system 112 including a tracking module 114 a plurality of reference sensors 116, and a transmitter mat 118 (also called an EM filed generator); and a computing device 120 including software and/or hardware used to facilitate pathway planning, identification of target tissue, navigation to target tissue, confirmation of placement of an extended working channel 102, or a suitable device there through (e.g., catheter 10), relative to a target (e.g., a nerve 40 FIG. 2) and monitoring the application of microwave energy into a target.

Continuing with reference to FIG. 3, system 100 further includes a catheter 10 insertable into the extended working channel 102 to access a target. The catheter 10, as described above, may include a microwave ablation antenna 16 which is coupled to a microwave generator 122. In one embodiment, the microwave ablation antenna 16 is also coupled to radiometer 124 which is useable to detect changes in the condition of the tissue. Although radiometer 124 is illustrated as being a separate component from microwave ablation antenna 16, in embodiments, radiometer 124 may be incorporated into microwave ablation antenna 16 or the microwave generator 122. Microwave generator 122 is configured to supply microwave energy to the microwave ablation antenna 16 to ablate a target or treat a target in the manner described above. An exemplary microwave generator 122 is the EMPRINT™ Microwave Ablation System currently sold by Medtronic plc.

Computing device 120 may be any suitable computing device including a processor and storage medium, wherein the processor is capable of executing instructions stored on the storage medium. The computing device 120 may further include a database configured to store patient data, CT data sets including CT images, navigation plans, and any other such data. Although not explicitly illustrated, the computing device 120 may include inputs, or may otherwise be configured to receive, CT data sets and other data described herein. Additionally, computing device 120 includes a display configured to display graphical user interfaces. Computing device 120 may be connected to one or more networks through which one or more databases may be accessed.

With respect to a pathway planning phase, computing device 120 utilizes computed tomographic (CT) image data for generating and viewing a three-dimensional model of the patient's "P's" airways, enables the identification of a target on the three-dimensional model (automatically, semi-automatically, or manually), and allows for determining a pathway through the patient's "P's" airways to the target. More specifically, the CT scans are processed and assembled into a three-dimensional CT volume, which is then utilized to generate a three-dimensional model of the patient's "P's" airways. The three-dimensional model may be displayed on a display associated with computing device 120, or in any other suitable fashion. Using computing device 120, various views of the three-dimensional model or two-dimensional images generated from the three-dimensional model are presented. The three-dimensional model may be manipulated to facilitate identification of target on the three-dimensional model or two-dimensional images, and selection of a suitable pathway through the patient's "P's" airways to access the target can be made. Once selected, the pathway plan, 3D model, and images derived therefrom can be saved and exported to a navigation system for use during the navigation phase(s). One such planning software is the ILOGIC® planning suite currently sold by Medtronic pic.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments.

Throughout this description, the term "proximal" refers to the portion of the device or component thereof that is closer to the clinician and the term "distal" refers to the portion of the device or component thereof that is farther from the clinician. Additionally, in the drawings and in the description above, terms such as front, rear, upper, lower, top, bottom, and similar directional terms are used simply for convenience of description and are not intended to limit the disclosure. In the description hereinabove, well-known functions or constructions are not described in detail to avoid obscuring the disclosure in unnecessary detail.

The invention is described by the following numbered paragraphs: -
1. A microwave ablation device comprising:
   a catheter having an inner tube defining an inflow lumen therein and an outflow lumen between the inner tube and an inner surface of the catheter;
   a balloon disposed on a distal portion of the catheter and in fluid communication with the inflow and outflow lumens;
   a microwave ablation antenna, insertable into one of the inflow or outflow lumens; and
   at least one temperature sensor for detecting a temperature of at least one of a fluid circulating in the catheter or an airway wall against which a portion of the catheter is positioned.
2. The microwave ablation device of paragraph 1, further comprising at least one inflow port in fluid communication with the inflow lumen and for receiving inflow of a fluid.
3. The microwave ablation device of paragraph 1, further comprising at least one outflow port in fluid communication with the outflow lumen and for allowing outflow of a fluid.
4. The microwave ablation device of paragraph 1, further comprising a port for receiving the microwave ablation antenna and limiting loss of any fluid in the catheter.
5. The microwave ablation device of paragraph 1, wherein inflation of the balloon is achieved by a fluid introduced therein.
6. The microwave ablation device of paragraph 1, further comprising at least one position sensor operably coupled to at least one of a distal portion of the catheter or the balloon.
7. The microwave ablation device of paragraph 6, wherein the at least one position sensor is an electromagnetic sensor.
8. A system comprising:
   a catheter having an inner tube defining an inflow lumen therein and an outflow lumen between the inner tube and an inner surface of the catheter;
   a balloon disposed on a distal portion of the catheter and in fluid communication with the inflow and outflow lumens;
   a microwave ablation antenna insertable into the catheter for placement within the balloon; and
   a microwave generator in electrical communication with the microwave ablation antenna.
9. The system of paragraph 8, further comprising at least one temperature sensor for detecting a temperature of at least one of a fluid in the balloon or an airway wall against which a portion of the catheter is positioned.
10. The system of paragraph 9, wherein upon detecting a predetermined temperature of a fluid within the balloon the microwave generator is switched off.
11. The system of paragraph 9, wherein upon detecting a predetermined rate of change of temperature of a fluid within the balloon the microwave generator is switched off.
12. The system of paragraph 8, further comprising an algorithm for driving the microwave generator to achieve a desired temperature profile of a fluid in the balloon or a desired temperature profile of an airway wall against which a portion of the catheter is positioned to limit damage to an airway in which the catheter is placed.
13. The system of paragraph 8, further comprising at least one electromagnetic sensor secured to the catheter.
14. The system of paragraph 13, further comprising an electromagnetic navigation system for detecting a position of the electromagnetic sensor.
15. The system of paragraph 14, wherein the electromagnetic navigation system permits placement of the catheter and microwave ablation antenna proximate a desired location within lungs of a patient such that application of energy results in severing of a nerve and denervation of tissue at the desired location.
16. A system comprising:
   a microwave ablation antenna; and
   a catheter configured to receive the microwave ablation antenna, the catheter including:
      an inner tube defining an inflow lumen and an outflow lumen, the outflow lumen defined between the inner tube and an inner surface of the catheter;
      a balloon disposed on a distal portion of the catheter and in fluid communication with the inflow and outflow lumens, the balloon configured to receive a fluid and surround a distal portion of the microwave ablation antenna when the catheter receives the microwave ablation antenna; and
      a temperature sensor operably coupled to the balloon and configured to detect a temperature of at least one of the fluid received by the balloon or an airway wall against which a portion of the balloon is positioned.
17. The system of paragraph 16, further comprising a microwave generator configured to deliver microwave energy to the microwave ablation antenna.
18. The system of paragraph 17, wherein the microwave generator is configured to be driven by an algorithm to achieve a desired temperature profile of the fluid received by the balloon or a desired temperature profile of the airway wall against which a portion of the balloon is positioned to limit damage to an airway in which the catheter is placed.
19. The system of paragraph 16, further comprising at least one electromagnetic sensor operably coupled to at least one of the microwave ablation antenna or the catheter.
20. The system of paragraph 19, further comprising an electromagnetic navigation system for detecting a position of the electromagnetic sensor.

## Claims

1. A microwave ablation device comprising:
a catheter having an inner tube defining an inflow lumen therein and an outflow lumen between the inner tube and an inner surface of the catheter;
a balloon disposed on a distal portion of the catheter and in fluid communication with the inflow and outflow lumens;
a microwave ablation antenna, insertable into one of the inflow or outflow lumens; and
at least one temperature sensor for detecting a temperature of at least one of a fluid circulating in the catheter or an airway wall against which a portion of the catheter is positioned.

2. The microwave ablation device of claim 1, further comprising at least one inflow port in fluid communication with the inflow lumen and for receiving inflow of a fluid.

3. The microwave ablation device of claim 1 or claim 2, further comprising at least one outflow port in fluid communication with the outflow lumen and for allowing outflow of a fluid; and/or further comprising a port for receiving the microwave ablation antenna and limiting loss of any fluid in the catheter.

4. The microwave ablation device of any preceding claim, wherein inflation of the balloon is achieved by a fluid introduced therein.

5. The microwave ablation device of any preceding claim, further comprising at least one position sensor operably coupled to at least one of a distal portion of the catheter or the balloon; preferably wherein the at least one position sensor is an electromagnetic sensor.

6. A system comprising:
a catheter having an inner tube defining an inflow lumen therein and an outflow lumen between the inner tube and an inner surface of the catheter;
a balloon disposed on a distal portion of the catheter and in fluid communication with the inflow and outflow lumens;
a microwave ablation antenna insertable into the catheter for placement within the balloon; and
a microwave generator in electrical communication with the microwave ablation antenna.

7. The system of claim 6, further comprising at least one temperature sensor for detecting a temperature of at least one of a fluid in the balloon or an airway wall against which a portion of the catheter is positioned.

8. The system of claim 6 or claim 7, wherein upon detecting a predetermined temperature of a fluid within the balloon the microwave generator is switched off.

9. The system of any of claims 6 to 8, wherein upon detecting a predetermined rate of change of temperature of a fluid within the balloon the microwave generator is switched off.

10. The system of any of claims 6 to 9, further comprising an algorithm for driving the microwave generator to achieve a desired temperature profile of a fluid in the balloon or a desired temperature profile of an airway wall against which a portion of the catheter is positioned to limit damage to an airway in which the catheter is placed.

11. The system of any of claims 6 to 10, further comprising at least one electromagnetic sensor secured to the catheter; preferably further comprising an electromagnetic navigation system for detecting a position of the electromagnetic sensor; further still wherein the electromagnetic navigation system permits placement of the catheter and microwave ablation antenna proximate a desired location within lungs of a patient such that application of energy results in severing of a nerve and denervation of tissue at the desired location.

12. A system comprising:
a microwave ablation antenna; and
a catheter configured to receive the microwave ablation antenna, the catheter including:
an inner tube defining an inflow lumen and an outflow lumen, the outflow lumen defined between the inner tube and an inner surface of the catheter;
a balloon disposed on a distal portion of the catheter and in fluid communication with the inflow and outflow lumens, the balloon configured to receive a fluid and surround a distal portion of the microwave ablation antenna when the catheter receives the microwave ablation antenna; and
a temperature sensor operably coupled to the balloon and configured to detect a temperature of at least one of the fluid received by the balloon or an airway wall against which a portion of the balloon is positioned.

13. The system of claim 12, further comprising a microwave generator configured to deliver microwave energy to the microwave ablation antenna; preferably wherein the microwave generator is configured to be driven by an algorithm to achieve a desired temperature profile of the fluid received by the balloon or a desired temperature profile of the airway wall against which a portion of the balloon is positioned to limit damage to an airway in which the catheter is placed.

14. The system of claim 12 or claim 13, further comprising at least one electromagnetic sensor operably coupled to at least one of the microwave ablation antenna or the catheter.

15. The system of claim 14, further comprising an electromagnetic navigation system for detecting a position of the electromagnetic sensor.
